# EUROPEAN PATENT APPLICATION

(11) **EP 3 954 367 A1**
(43) Date of publication of application: **16.02.2022**
(21) Application number: 21000228.3
(22) Date of filing: 11.08.2021
(51) Int. Cl.: A61K 31/164, A61K 31/355, A61K 31/385, A61K 31/4415, A61K 31/455, A61K 31/51, A61K 31/714, A61K 33/08, A61K 33/30, A61K 38/00, A61K 47/00, A61P 25/02, A61P 25/04

(54) **DIETARY SUPPLEMENT FOR THE PREVENTION AND/OR TREATMENT OF PAIN AND/OR NEUROPATHIES**

(30) Priority: 13.08.2020 GR 20200100479
(71) Applicant: Pharma Unimedis PC, 15127 Melissia Attikis (GR)
(72) Inventor: Tsantes, Dimitrios, GR - 15127 Melissia Attikis (GR)
(74) Representative: Georgakopoulou, Pavlina

(57) **Abstract**

The present invention relates to a pharmaceutical composition or dietary supplement composition or food for special medical purposes for the prevention and/or treatment of neuropathies and/or pain comprising a therapeutically effective amount of Palmitoylethanolamide or a pharmaceutically acceptable salt or derivative thereof in combination with a therapeutically effective amount of alpha-lipoic acid and at least another one compound such as superoxide dismutase to be used for the prevention and/or treatment of pain and/or neuropathies, and a process for the preparation of said composition.

## Description

### TECHNICAL FIELD OF THE INVENTION

The present invention relates to a pharmaceutical composition or dietary supplement composition or food for special medical purposes for the prevention and/or treatment of neuropathies and/or pain in general, and in particular the present invention relates to a pharmaceutical or dietary supplement composition or food for special medical purposes comprising a therapeutically effective amount of Palmitoylethanolamide or a pharmaceutically acceptable salt or derivative thereof in combination with a therapeutically effective amount of alpha-lipoic acid and at least another one compound to be used for the prevention and/or treatment of pain and/or neuropathies, and a process for the preparation of said composition.

### BACKGROUND OF THE INVENTION

Neuropathy is a condition that results from damage to, or dysfunction of, the nervous system. Most often, the damage exists in the peripheral nervous system, which lies beyond the spine and brain, although brain injury, such as stroke, can also result in neuropathic symptoms.

The symptoms of neuropathy depend on the underlying nerves whose function has been affected. Neuropathy that damages sensory nerves can cause numbness, weakness and stabbing or burning pain - symptoms that may worsen if not treated early. If there has also been damage to the type of nerves that convey the sense of touch, vibration, and temperature, patients may experience tingling, numbness, or the sense of wearing an invisible glove or sock over their hands or feet. If there is damage to motor nerves that control stability and movement, patients may have a lack of coordination, weakness, or cramping.

Neuropathy is a leading cause of chronic pain, which persists for three months or more. Several people who report chronic pain suffer from some form of neuropathy. Pain that falls under the broad category of a pain of neuropathic origin includes neuralgia, or neuritis among others. Neuritis is caused by inflammation of a nerve or group of nerves and may be accompanied by other symptoms, such as fever and swelling.

While some neuropathies occur because of damage to peripheral (small) nerves and nerve endings, other types of neuropathic pain happen after an injury in the central nervous system (brain and/or spine). These neuropathic pain conditions that arise in the brain or spine are called central pain syndromes. One example of a central pain syndrome is post-stroke shoulder pain, which is estimated to occur in up to one-third of stroke survivors.

Therefore, regardless of where the original nerve injury occurred, in many instances, the central nervous system can play a role in the continued experience of chronic pain symptoms.

Peripheral neuropathy refers to the many conditions that involve damage to the peripheral nervous system, the vast communication network that sends signals between the central nervous system (the brain and spinal cord) and all other parts of the body. Peripheral nerves send many types of sensory information to the central nervous system (CNS), such as a message that the feet are cold. They also carry signals from the CNS to the rest of the body.

Neuropathy is very common. The condition affects people of all ages; however, older people are at increased risk. Other than age, some of the more common risk factors for neuropathy include diabetes, metabolic syndrome (high blood pressure, high cholesterol, obesity, diabetes), and heavy alcohol use. People in certain professions, such as those that require repetitive motions, have a greater chance of developing mononeuropathies from trauma or compression of nerves.

Neuropathic Pain (also known as nerve pain) is a complex, chronic pain state that usually is accompanied by tissue injury. With neuropathic pain, the nerve fibers themselves might be damaged, dysfunctional, or injured. These damaged nerve fibers send incorrect signals to other pain centers. The impact of a nerve fiber injury includes a change in nerve function both at the site of injury and areas around the injury.

Multiple causes of neuropathic pain have been described and its incidence is likely to increase due to the ageing of the population, increased incidence of diabetes mellitus and improved survival from cancer after chemotherapy. The burden of chronic neuropathic pain seems to be related to the complexity of neuropathic symptoms, poor outcomes and difficult treatment decisions. Many patients with neuropathic pain may experience anxiety, depression, insomnia, disability, and reduced quality of life.

Pharmacologic management focuses on neuropathic pain as a broad clinical entity, and regardless of cause, similar treatment strategies often are used. Pharmacotherapy remains an important modality for the treatment of neuropathic pain. However, as monotherapy current drugs are associated with limited efficacy and dose-related side effects. Combining two or more different drugs may improve analgesic efficacy and, in some situations, reduce overall side effects (e.g. if synergistic interactions allow for dose reductions of combined drugs). Unfortunately, treatment still remains suboptimal because traditional drugs provide only modest pain relief and neuropathic pain remains difficult to treat, challenging providers across multiple disciplines.

There are number of treatments that can relieve the pain, inflammation, and discomfort associated with neuropathies. One treatment involves the use of non-steroidal anti-inflammatory drugs (NSAIDs) such as aspirin and ibuprofen. Although NSAIDs relieve some stiffness, inflammation, and pain, NSAIDs can lead to side effects such as gastric bleeding, liver damage, and kidney damage. In addition, long-term use of NSAIDs can lead to reduced effectiveness. Further, the treatment is restricted to alleviation of the symptoms and does nothing to improve the underlying condition.

Therefore, despite the progress in the understanding of the pathophysiology of pain, neuropathy and neuropathic pain and the development of new treatment procedures, there is a need for a multidisciplinary approach to the management of pain, neuropathic pain and neuropathies in general and a medicinal treatment and/or prevention that is effective and well tolerated without undesirable side effects.

### SUMMARY OF THE INVENTION

It is, therefore, an object of the present invention to provide a pharmaceutical or dietary supplement composition or food for special medical purposes for the prevention and/or treatment of pain and/or neuropathies, which overcomes the deficiencies of the prior art.

It is another object of the present invention to provide a pharmaceutical or dietary supplement composition or food for special medical purposes for the prevention and/or treatment of pain and/or neuropathies, which is safe and effective with no side effects and good pharmacotechnical properties.

Moreover, it is another object of the present invention to provide a suitable process for the preparation of a pharmaceutical or dietary supplement composition for oral administration or food for special medical purposes, which is effective and reproducible.

In accordance with the above objects of the present invention, a pharmaceutical or dietary supplement composition or food for special medical purposes is provided comprising a therapeutically effective amount of palmitoylethanolamide or a pharmaceutically acceptable salt or derivative thereof, in combination with a therapeutically effective amount of alpha-lipoic acid and at least one additional compound for the prevention and/or treatment of pain and/or neuropathies.

According to another embodiment of the present invention, a process for the preparation of a pharmaceutical or dietary supplement composition or food for special medical purposes comprising a therapeutically effective amount of palmitoylethanolamide (PEA) or a pharmaceutically acceptable salt or derivative thereof, in combination with a therapeutically effective amount of alpha-lipoic acid (ALA) and at least one additional compound for the prevention and/or treatment of pain and/or neuropathies is provided, wherein said process comprises following steps: a) Mixing the individual active substances PEA, ALA and/or the at least one additional compound with tableting excipients such as binder, filler, lubricant and/or glidant in order to obtain uniform agglomeration; b) Adding all said agglomerates of the active substances from step a) in a blender and mixing for appropriate time so as to form a homogenous mixture; c) Sieving the obtained mixture of step b) and adding to the sieved mixture the total amount of at least one additional ingredient such as vitamins and/or minerals and at least one optional additional tableting excipient such as binder, diluent, lubricant and/or glidant and mixing for appropriate time until uniform; d) Compressing the final blend from step c) into a desired tablet form, and e) Optionally applying to the tablet a gastro-resistant or enteric film coating by any conventional method.

Further preferred embodiments of the present invention are defined in dependent claims 2 to 10 and 12 to 14.

Other objects and advantages of the present invention will become apparent to those skilled in the art in view of the following detailed description.

### DETAILED DESCRIPTION OF THE INVENTION

It has been surprisingly found that the object of the present invention is achieved by employing Palmitoylethanolamide due to its anti-inflammatory and analgesic properties, in combination with Alpha-lipoic acid and at least one additional compound such as peroxide dismutase, which are potent antioxidants for the treatment and/or prevention of pain and /or neuropathies.

Palmitoylethanolamide, also known as PEA, N-Palmitoylethanolamine, N-(2-hydroxyethyl) hexadecanamide or Palmidrol is an amide deriving from palmitic acid and ethanolamine. It belongs to the N-acylethanolamine family, which also includes anandamide, the endogenous ligand of the cannabinoid and oleoylethanolamide receptors. PEA was identified for the first time in the 1950s, as an anti-inflammatory compound isolated in egg yolk. Preclinical and clinical studies suggest that the PEA might potentially be useful in treatment of various pathologies, such as eczema and various types of pain and, at the same time to be essentially free of undesired effects in humans.

Palmitoylethanolamide (PEA) is a lipidic mediator known to exert antinociceptive effects against different types of pain and is characterized by an interesting antineuropathic profile, especially when used in a micronized or ultra-micronized form. Palmitoylethanolamide (PEA), as used in the composition of the present invention, may be in non-micronized form, in micronized form, in ultra-micronized form or mixtures thereof.

There exist various mechanisms, which explain the pharmacological action of PEA. This substance can, in fact, act in a direct or indirect manner on various receptors such as receptors of the endocannabinoid system. Palmitoylethanolamide can be useful in the treatment of diseases associated with or resulting from neurodegeneration, hyperalgesia and inflammation, as well as brain damage resulting from ischemia. PEA also has a protective effect on neurological disorders induced by cerebral ischemia and traumatic brain damage. In these conditions, in fact, an increase in tissue levels of PEA in the central nervous system is often observed, since this molecule has the function of protecting neurons from damage induced by ischemia and brain trauma.

The composition of the present invention further comprises Alpha-lipoic acid (ALA), which is an organic compound in the body that acts as a powerful antioxidant and is therefore widely used in clinical practice. It may have several health benefits. A few studies have suggested that ALA may help reduce the symptoms of peripheral neuropathy - nerve damage that can be caused by diabetes. However, alpha-lipoic acid is an active ingredient that has some limitations with regard to its bioavailability and its metabolism. Some studies have shown that ALA is absorbed quickly and almost completely, if administered orally, but has a rather limited bioavailability (about 30%) due to a high pre-systemic metabolism. The central problem in the use of ALA is therefore to improve their bioavailability in quantitative terms and to reduce the inter-individual variability in gastro-intestinal absorption. Furthermore, food intake reduces the absorption of ALA, hence it is suggested to take it on empty stomach. Another intrinsic limit to this molecule is its instability, as ALA can polymerize.

Alpha-lipoic acid is an antioxidant made by the body and it is found in every cell, where it helps turn glucose into energy. Antioxidants attack "free radicals," waste products created when the body turns food into energy. Free radicals cause harmful chemical reactions that can damage cells, making it harder for the body to fight off infections. They also damage organs and tissues. Several studies suggest alpha-lipoic acid helps lower blood sugar levels. Its ability to kill free radicals may help people with diabetic peripheral neuropathy, who have pain, burning, itching, tingling, and numbness in arms and legs from nerve damage. Researchers believe Alpha-lipoic acid also helps to improve insulin sensitivity.

Some antioxidants are water soluble, such as vitamin C and other antioxidants are fat soluble such as vitamin E. However, alpha-lipoic acid is both fat and water soluble, i.e. it can work throughout the body.

The composition of the present invention further comprises superoxide dismutase (SOD), a key cellular antioxidant, which is highly responsible for the elimination of O₂. Superoxide dismutases (SODs) constitute a very important antioxidant defense against oxidative stress in the body.

SOD is an enzyme, which destroys harmful superoxides (active oxygen molecules), and is present in all organs and intracellular fractions (cytoplasm and mitchondria). Although the enzyme called extracellular SOD is present in the blood, its activity is very low, and therefore, its defense mechanism in the cell membrane and in the space outside of tissue cells is very weak, and then oxidative disorders highly risky to an organism sometimes occur in these local sites. The enzyme SOD acts as a good therapeutic agent against reactive oxygen species-mediated diseases. Many studies have revealed the critical role of oxidative stress in carcinogenesis. Indeed, there are several clear evidences indicating that reactive oxygen species (ROS) work as endogenous class of carcinogens by inducing mutations in the cells. It has been suggested that SOD may regulate cancer progression and, hence, can be used as a novel target for cancer treatment. Furthermore, it has been shown that Cu, Zn-SOD can be used as a novel therapeutic target for the treatment of multiple myeloma.

Several studies have been performed that revealed the therapeutic potential and physiological importance of SOD. The enzyme can serve as an anti-inflammatory agent and can also prevent precancerous cell changes. Natural SOD levels in the body drop as the body ages and hence as one age, one becomes more prone to oxidative stress-related diseases. However, SOD has certain limitations in clinical applications due to an in vivo instability and short half-life thereof.

In order to prolong the half-life of SOD in the blood, various chemical modifications have been made. For example, modifications of SOD with polyethylene glycol, with high molecular weight dextran, with inulin or the like. These modifications have still various drawbacks and when modified SODs are modified with agents which are not or rarely present in nature, an administration of modified SODs can give rise to side effects.

In one embodiment, the composition according to the present invention comprises, in addition to active ingredients PEA, ALA and SOD, at least one further active ingredient of natural or synthetic origin with therapeutic action, or supplementary action, or otherwise useful for the proposed purposes of the present invention. Non- limiting examples of said active ingredients are vitamins and/or minerals, such as the B complex vitamins, in particular thiamine, vitamin PP; minerals, such as Zinc and Magnesium; antioxidants, such as vitamin C and vitamin E; neurotrophic and/or nootropic agents, such as citicoline, acetyl-L- carnitine, phosphatidylserine, phosphatidylcholine and plant extracts of the genera Panax and Bacopa.

The composition of the present invention further may comprise vitamins and/or minerals, as vitamins and minerals can help reduce inflammation and rebuild joint cartilage. Examples of vitamins and minerals including, without limitation, pyridoxine chloride, gluthione, calcium citrate, magnesium citrate, magnesium oxide, calcium carbonate (e.g., lead-free calcium carbonate), ascorbic acid, zinc acetate, zinc gluconate and vitamin B complexes.

The B complex vitamins are a group of water-soluble organic compounds, which while structurally diverse, all play crucial roles in the maintenance of body functions, including proper cellular function, tissue growth and development. The B vitamin family includes vitamin B1 (thiamine), vitamin B2 (riboflavin), vitamin B3 (niacin), vitamin B5 (pantothenic acid), vitamin B6 (pyridoxine), vitamin B9 (folate), and vitamin B12 (cobalamin). With the exception of vitamin B3, which can be synthesized from tryptophan, all of the B vitamins the human body requires must be provided from a dietary or exogenous source. It is already known that the B complex vitamins (B1, B6 and B12) play fundamental roles in the nervous system functions and contribute to promote nerve repair, both in acceleration of nerve tissue regeneration and recovery of nerve function by a variety of mechanism.

The B vitamins may also be employed for pain relief and hyperalgesia. In addition to neurological impact of B vitamin deficiencies, vitamins B1, B6 and B12 have been shown to exert anti-inflammatory and analgesic effects in animal models. The B complex vitamins, especially vitamin B12 and vitamin B6 (pyridoxine), are powerful immune-boosting agents and protect against microbial and viral infections.

Further, the composition of the present invention may comprise vitamin PP (also known as Vitamin B3, nicotinic acid, or niacin) which plays a key role in the metabolism of proteins, fats and carbohydrates into energy. In addition, vitamin PP is purported to promote a number of other health benefits including, for example, possibly decreasing the risk of coronary artery disease. Vitamin PP is present in a number of natural sources including, for example, meats and fortified grain products. Other B vitamins as well as additional substances may further promote these beneficial health effects.

Vitamin E or α-tocopherol is a fat-soluble vitamin that has antioxidant activity. This vitamin is lipophilic so in the composition according to the present invention its purpose is to increase lipid production at neuronal membrane level, the first targets of free radicals and it is particularly useful in protecting membranes from lipid peroxidation. This means that vitamin E acts as a key defense mechanism against oxidation reactions.

Vitamin E is found in cell membranes, where it reacts with active oxygen compounds (free radicals) and inactivates them before they oxidize unsaturated membrane lipids and destroy their structure. In this way it slows down the destruction of biological membranes and other biomolecules by free radicals. Its role in cellular defense against oxygen free radicals is crucial as its deficiency appears to cause oxidative damage to cells resulting in pathological conditions related to respiratory function and the immune system (eg emphysema, bronchitis).

Vitamin E seems to reduce and even prevent the occurrence of chronic diseases. In particular, vitamin E is likely to act as an antioxidant or directly in the functioning of the immune system, reducing respiratory inflammation. For example, it helps reduce the severity of asthma and even prevent episodes. Vitamin E appears to play a protective role in allergic bronchial asthma by regulating the activity of macrophages, the cells that proliferate in the lungs and are involved in the pathogenesis of asthma.

Vitamin E also appears to affect the regulation of dendritic cell maturation, which are the antigen-presenting cells of the immune system in order to locate the site of infection. Vitamin E has been shown to regulate T-cell activation at an early stage, increasing their proliferation. In this way, vitamin E is involved in protection against pathogenic microorganisms and the development of chronic diseases as it stimulates T-cells which are involved in allergic and inflammatory reactions. Also, daily intake of vitamin E seems to improve the immune system's B-cell response to antibody production.

Vitamin E is absorbed in the small intestine, then passes to the liver and from there is transported through the bloodstream to the places where it should act as an antioxidant. Most of it is stored in adipose tissue, liver and muscle. Vitamin E deficiency can cause: Emphysema, Bronchitis, Neurological disorders, Ataxia (disturbance of balance and coordination), Peripheral neuropathy, Myopathy, Anemia. In conclusion, daily intake of vitamin E seems to improve lung function and strengthen the immune system.

In addition, the composition of the present invention may contain more than one vitamin. For example, the composition may contain two different vitamins. Likewise, the composition of the present invention may contain more than one mineral. For example, the composition of the present invention may contain two different minerals, such as Zinc and Magnesium.

Zinc is an essential trace mineral that plays an important role such as growth, immune response, gene expression, wound healing, neurological function. Zinc ion levels are strictly maintained by physiologic mechanisms. Transport of zinc ion requires specific zinc transporter proteins because zinc can't pass cellular membrane. Moreover, it is considered that the alteration of zinc concentration modulates pain signaling.

The presence of zinc (Zn) further enhances the antimicrobial and antiviral activity of the composition of the present invention. Zinc (Zn) is the second most abundant metal in the human body with anti-inflammatory and antioxidant properties. It is an essential trace element for basic cellular processes, such as cell growth, differentiation and survival.

The role of zinc as a cofactor in more than 300 enzymes and as a regulator of proteins, is crucial for the proper functioning of cells. In fact, about 10% of the proteins encoded by the human gene and involved in cellular activity can bind zinc to different structural regions. Zinc acts as a cofactor in proteins and affects the structural and catalytic functions of enzymes and transcription factors. It may also act as a second signaling molecule in signaling pathways of the immune system.

In addition, zinc can act as a neurotransmitter in neuronal communication, but also as a regulator in the action of hormones, growth factors, and cytokines by binding to them. Zinc has a signal role in regulating the hypersensitivity reaction, i.e. it activates the initiation of the allergic response of the immune system. In addition, zinc is involved in the activation of dendritic cells during the immune response, pro-inflammatory response, cell survival and antibody production.

Zinc affects the immune system's response to various infections. Zinc supplements appear to improve immunity as well as chronic inflammatory responses. Therefore, zinc is essential for maintaining cellular homeostasis and function.

In addition, zinc activates T-cells that act as helpers on B-cells to induce the production of antibodies and the death of pathogenic-infected cells. Zinc appears to play a role in signaling the growth and maturation of B-cells. This means that it participates in the control of homeostasis and the function of lymphocytes. Zinc is also related to the response of the immune system to pathogens that cause respiratory disorders (e.g. pneumonia). In particular, it seems to improve the function of the immune system, thereby increasing resistance to pathogens and reducing the use and duration of antimicrobial therapy. In fact, zinc supplements reduce morbidity and mortality due to pneumonia in the elderly.

Zinc supplements significantly improve the life expectancy and quality of life of the elderly with diseases of the respiratory system. Zinc intake appears to significantly reduce the risk of infections and therefore helps reduce the number and duration of pneumonia episodes, as well as the number of antibiotics and the duration of their illness.

Zinc supplements play an important role in the development of atopic asthma. Specifically, by influencing regulators of the immune response (interferons, interleukins, T-lymphocytes) and reducing oxidative stress, they improve atopic asthma. Zinc contributes to the effective reduction of the severity and duration of the symptoms of colds from viruses (e.g. rhinovirus, coronavirus, adenovirus), as it reduces the activation of inflammatory factors (monocytes - macrophages) and oxidative stress.

Zinc is well established as an essential trace element and its metabolic role in the nervous system has received increasing research attention in recent years. Most of the reported research related to zinc has been concerned with the central nervous system.

Zinc deficiency appears to increase oxidative stress and disrupt immune system function while it can cause: Recession of the immune response, Hypersensitivity reactions (allergies), Autoimmune Diseases, Atrophy of the thymus gland, Lymphopenia, Increase the duration of inflammation, Enteropathic acrodermatitis, Alzheimer's, Hypertension, Diabetes, Rheumatoid arthritis, Multiple sclerosis. Zinc is ingested exogenously from food or dietary supplements, absorbed in the small intestine and from there released into the bloodstream. Further, Zinc as a key antioxidant and antimicrobial agent seems to protect against respiratory infections and improve the immune system.

Magnesium is very important mineral for the normal functioning of cells, nerves, muscles, bones, and heart. From a neurological standpoint, magnesium plays an essential role in nerve transmission and neuromuscular conduction. It also functions in a protective role against excessive excitation that can lead to neuronal cell death (excitotoxicity), and has been implicated in multiple neurological disorders. Due to these important functions within the nervous system, magnesium is a mineral of intense interest for the potential prevention and treatment of neurological disorders.

Magnesium is essential for regulation of muscle contraction (including that of the heart), blood pressure, insulin metabolism, and is required for the synthesis of DNA, RNA, and proteins. In the nervous system, magnesium is important for optimal nerve transmission and neuromuscular coordination, as well as serving to protect against excitotoxicity (excessive excitation leading to cell death).

One of the main neurological functions of magnesium is due to magnesium's interaction with the *N*-methyl-d-aspartate (NMDA) receptor. Magnesium serves as a blockade to the calcium channel in the NMDA receptor, and must be removed for glutamatergic excitatory signaling to occur. Low magnesium levels may theoretically potentiate glutamatergic neurotransmission, leading to a supportive environment for excitotoxicity, which can lead to oxidative stress and neuronal cell death. Abnormal glutamatergic neurotransmission has been implicated in many neurological and psychiatric disorders including: migraine, chronic pain, epilepsy, Alzheimer's, Parkinson's, and stroke, in addition to depression and anxiety, which are commonly comorbid with these neurological disorders. Molecular studies and animal studies have shown neuronal protection from pre-treatment with magnesium, making this mineral of intense interest for its potential neuroprotective role in humans. Thus, magnesium could be an important dietary factor in the prevention and/or treatment of the above conditions. Moreover, clinical experience, as well as research in nerve pain conditions such as pancreatic cancer, has shown that magnesium can be an effective treatment for pain.

The composition of the present invention may comprise magnesium oxide, which is a type of magnesium mineral supplement that it actually contains more magnesium than other magnesium supplements. Magnesium oxide has many health benefits. When used regularly, magnesium oxide can help boost low magnesium levels, relieve constipation, manage depression, treat migraines, and more.

Magnesium deficiency has a number of contributing factors including diet, food allergies, alcoholism, and poor kidney health. Symptoms of magnesium deficiency may include: leg cramps, muscle and bone pain, anxiety and depressed moods, high blood pressure, insomnia, fatigue. Supplementation of magnesium oxide can maintain healthy levels of magnesium throughout the body. In fact, supplementation eliminates many of the symptoms associated with deficiency.

In the composition according to the present invention, preferably PAE is comprised in an amount by weight between 20 % and 40% of the total weight of the composition, preferably between 25% and 35%, ALA is present in an amount by weight comprised between 20% and 40% of the total weight of the composition, preferably between 25% and 35% and/or SOD is present in an amount by weight comprised between 1% and 10%, preferably between 4% and 8% by weight/total weight of the composition. The composition of the present invention may contain any amount of vitamins and minerals according to the Recommended daily allowance (RDA).

In addition, the composition of the present invention can beneficially provide a user with a significant fraction of the RDA of various nutritional components contained therein.

The pharmaceutical compositions of the present invention may also contain one or more additional formulation ingredients selected from a wide variety of excipients. According to the desired properties of the composition, any number of ingredients may be selected, alone or in combination, based upon their known uses in preparation of dietary supplement compositions. Moreover, any excipient may optionally be added to the above composition, provided that they are compatible with the active ingredients of the composition, in order to overcome problems associated with unfavorable pharmacotechnical characteristics of these substances, and in order to increase the stability of the drug and the shelf-life of the pharmaceutical product.

The composition of the present invention may contain one or more radical scavengers, antioxidants, reducing agents, or mixtures thereof. Typically, a dietary supplement contains one or more radical scavengers, antioxidants, reducing agents, or mixtures thereof in an amount that effectively reduces oxidation or degradation of the components of the composition. Examples of radical scavengers and antioxidants include, without limitation, ascorbic acid, tocopheryl acetate, tocopheryl palmitate, tocopherol, and butyl hydroxytoluene.

The pharmaceutical or dietary supplement composition or food for special medical purposes of the present invention may be in the form of a liquid, solution, suspension, pill, capsule, tablet, gel cap, powder, or gel. For oral administration, tablets or capsules may be prepared by conventional means with acceptable excipients such as binders (for example pregelatinized corn starch, polyvinylpyrrolidone or hydroxypropyl methylcellulose), fillers (for example lactose, microcrystalline cellulose or calcium hydrogen phosphate), lubricants (for example magnesium stearate, talc or silica), disintegrants, wetting agents, diluents, absorbents, adsorbents, glidants, colourants, surfactants, antioxidants, sweeteners, flavourings, plasticisers, viscosity enhancers, emulsifiers, preservatives and chelating agent and the like. The tablets may be coated, if desired. Liquid preparations for oral administration may take the form of, for example, solutions, syrups, or suspension, or they may be presented as a dry product for constitution with saline or other suitable liquid vehicle before use.

Dietary supplement compositions of the present invention also may contain acceptable additives as will be understood by one skilled in the art depending on the particular form of the dietary composition. The preparations for oral administration also may be suitably formulated to give delayed, controlled or extended release of the active ingredients.

In some cases, the composition according to the present invention may include one or more fillers or thickeners selected from the following: a hydroxyl containing compound, a dextrin or dextrin derivative, carboxymethyl cellulose, hydroxypropyl cellulose, hydroxyethyl cellulose, hydroxypropyl methyl cellulose, methyl cellulose, calcium caseinate, konjac, collagen, inulin, casein, wheat gluten, carrageenan, alginates, propylene glycol alginate, xanthan, dextrin, pullulan, curdlan, gellan, locust bean gum, guar gum, tara gum, gum tragacanth, pectin, agar, zein, karaya, gelatin, psyllium seed, chitin, chitosan, gum acacia, polyvinyl pyrrolidone, polyethylene oxide, polyvinyl alcohol, or a combination thereof. In some cases, the dietary supplement composition of the present invention can be substantially free of artificial colors, artificial flavors, and chemical preservatives.

The pharmaceutical or dietary composition or food for special medical purposes of the present invention will be prepared by conventional methods for obtaining tablets with modified release that are well known to the skilled person in pharmaceutical technology. The tablets according to the present invention may be coated by known techniques to delay disintegration and absorption in the gastrointestinal tract and thereby provide an extended action over a longer period. The tablets of the present invention may be made by compressing the active substances with one or more pharmaceutically acceptable ingredients. Compressed tablets may be prepared by compressing, in a suitable machine, the active substances in a free-flowing form such as a powder or granules, optionally mixed with a binder, lubricant, inert diluent, surface active, or dispensing agent.

Another embodiment of the present invention is the use of a process for the preparation of pharmaceutical or dietary supplement composition or food for special medical purposes for oral administration containing PEA or salt or derivative thereof and ALA and at least one additional active ingredient such as superoxide dismutase (SOD). The process steps may be as follows: Mixing the individual active substances PEA, ALA and/or the at least one additional compound with tableting excipients such as binder, filler, lubricant and/or glidant in order to obtain uniform agglomeration; Adding all said agglomerates of the active substances from step a) in a blender and mixing for appropriate time so as to form a homogenous mixture; Sieving the obtained mixture of step b) and adding to the sieved mixture the total amount of at least one additional ingredient such as vitamins and/or minerals and at least one optional additional tableting excipient such as binder, diluent, lubricant and/or glidant and mixing for appropriate time until uniform; Compressing the final blend from step c) into a desired tablet form, and Optionally applying to the tablet a gastro-resistant or enteric film coating by any conventional method.

All percentages stated herein are weight percentages based on total composition weight, unless otherwise stated.

The following examples illustrate preferred embodiments in accordance with the present invention without limiting the scope or spirit of the invention:

### EXAMPLES

### Example 1: Dietary Supplement Composition 1 of the present invention

Preferred pharmaceutical or dietary supplement composition 1 according to the present invention is illustrated in Table 1.

**TABLE 1:**

| | **Ingredients** | **% of total weight** |
|---|---|---|
| 1 | Palmitoylethanolamide (PEA) | 20 - 40 |
| 2 | Alpha lipoic-acid (ALA) | 20 - 40 |
| 3 | Superoxide dismutase 1 UI/mg (SOD) | 1 - 10 |
| 4 | Magnesium (from Magnesium oxide) | 1 - 5 |
| 5 | Zinc (from Zinc gluconate) | 0.1 - 2 |
| 6 | Vitamin E | 0.1 - 2 |
| 7 | Vitamin B12 | 0.05 - 2 |
| 8 | Vitamin B6 (from Pyridoxine hydrochloride) | 0.05 - 2 |
| 9 | Vitamin B1 (from Thiamine hydrochloride) | 0.05 - 2 |
| 10 | Vitamin PP | 0.05 - 2 |
| 11 | Microcrystalline Cellulose | 10 - 20 |
| 12 | Hydroxypropyl Cellulose | 0.5 - 5 |
| 13 | Hydroxypropyl Methylcellulose | 0.05 - 2 |
| 14 | Magnesium salts of fatty acids | 2-8 |
| 15 | Silicon dioxide | 0.05 - 2 |
| 16 | Talc | 0.05 - 2 |
| 17 | Coating | 0.05 - 2 |
| | **Total** | 100,00 |

Pharmaceutical or dietary supplement composition 1 of the present invention was prepared according to the following manufacturing process:
All ingredients were weighted and the individual active substances PEA, ALA and/or SOD were mixed with tableting excipients such as binder, filler etc. in order to form uniform agglomeration. Then, all said agglomerates of the active substances were added in a blender and mixed for appropriate time so as to form a homogenous mixture. To the mixture obtained the total amount of all remaining ingredients were added and finally mixed until uniform. The final mixture was then compressed into tablets of desired form in a tableting machine.

A coating solution was formed with the following excipients: Shellac, polyethylene glycol, hydroxypropyl methylcellulose, iron oxides and hydroxides, titanium dioxide. Subsequently, the tablets were coated with said enteric film coating. The enteric film coating was applied to the tablets by any conventional method.

Palmitoylethanolamide used in composition 1 of the present invention was in micronized or ultramicronized form. The ratio of palmitoylethanolamide to alpha-lipoic acid used in composition 1 of the present invention is from 1: 0.5 to 1:4, preferably from 1:1 to 1:3 and most preferably 1:1.

The bulk mixture showed satisfactory flow and the produced tablets were tested for hardness, friability, disintegration, and water content. All tests were performed according to European Pharmacopoeia and were well within the specifications.

Another object of the present invention was to prepare a pharmaceutical composition that is stable for a long period of storage time.

The pharmaceutical or dietary supplement composition of the present invention is specially designed to support patients suffering from chemotherapy-induced neuropathy (CIPN) and provides combined support of the nervous tissue, reduction of the painful symptoms and remission of the pain. Further, the pharmaceutical or dietary supplement composition of the present invention may be used for the prevention and/or treatment of the following diseases: a) Diabetic neuropathy: provides comprehensive etiological treatment of diabetic neuropathy, and due to vitamin B complex, Mg, Zn, SOD, ALA and Palmitoylethanolamide can lead to remission of symptoms and reduction of neuropathic pain; b) Osteoarthritis: Reduction of oxidative stress, improvement of functionality and remission of pain; c) Carpal tunnel syndrome: Improvement of clinical symptoms and -objective findings; d) Backache Pain: relief and improved mobility; e) Sciatica: Improving the neuropathic pain index and reducing the intake of analgesics; and f) Neck pain: Improving the subjective perception of pain and the feeling of physical well-being

The main goals achieved by the pharmaceutical or dietary supplement composition of the present invention for the prevention and/or treatment of the symptoms of neuropathy and chronic pain, regardless of the etiology, are divided into the following stages: a) Nervous tissue protection (before the onset of symptoms): the use of B complex vitamins, trace elements of magnesium and zinc according to the recommended daily allowance are proved to be powerful neurotrophic factors; b) Reduction of symptoms (pain, nausea, tingling, etc.): due to the presence of Palmitoylethanolamide (PEA), which is a potent analgesic and anti-inflammatory agent, and alpha-lipoic acid (ALA) and peroxide dismutase (SOD), which are potent antioxidants; and c) Quick repair of nerve tissue damage: this is achieved due to the presence of B complex vitamins, Alpha-lipoic acid, vitamin E, Nicotinamide in the pharmaceutical or dietary supplement composition of the present invention and said ingredients contribute to the faster recovery of nerve tissue.

The pharmaceutical or dietary supplement composition or food for special medical purposes of the present invention meets the management needs of patients with chronic pain and neuropathic symptoms at any stage and can contribute to a better outcome, prevention and rehabilitation. The content of the ingredients included in the pharmaceutical or dietary supplement composition of the present invention, in terms of activity-safety and especially the B complex vitamins and trace elements of Magnesium and zinc is in accordance with the recommended daily allowance RDA%.

Tablets prepared from the composition of the present invention are gastro-resistant film coated so that they are not destroyed by gastric acids and the active substances are available for absorption in the small intestine. Especially the SOD factor is in the form of GLISODIN^{®} (complex with wheat biopolymer) in order to maintain its structure during the passage through the digestive system and to be absorbed in the small intestine. Said tablets are gradual disintegration and sustained release and suitable for once daily administration. The recommeded daily dose is one or two tablets daily, depending on the recommndation of the doctor.

The composition of the present invention is lactose free. Due to the presence of fat-soluble and water-soluble vitamins, it is recommended to take it with food, in order to maximize absorption.

The composition of the present invention contains micronized & ultramicronized palmitoylethanolamide (PEA) in an ideal ratio in order to penetrate the cell membrane. Due to the high molecular weight, other forms of (PEA) are inactive.

The composition of the present invention can be also used as a food for special medical purposes that is specially designed to protect and prevent secondary complications due to chronic diseases. The food for special medical purposes of the present invention is suitable for the dietary management of patients with deficiency of certain endogenous substances such as vitamins, trace elements, antioxidants, saturated fatty acid amide. Endogenous substances are necessary to minimize pain and inflammation resulting from neural tissue damage (radiotherapy, chemotherapy, immunotherapy, surgical treatment, compressive neuralgia, diabetic neuropathies, etc.). Endogenous substances are necessary to protect cells from oxidative stress.

The composition of the present invention is a new treatment system specially designed to: Help for people with respiratory problems; Preventing the occurrence of lung diseases; Reducing the incidence of chronic lung disease; Strengthening the immune system.

The composition of the present invention preferably is available in tablet form and contains a set of endogenous factors (PEA, α-lipoic acid), vitamins (B1, B6, B12, B3, Vitamin E) and trace elements (magnesium, zinc) which are necessary to reduce pain and inflammation.

The composition of the present invention contains vitamin E and zinc in the recommended daily dose and is based on technology which ensures its slow release and gradual absorption in the small intestine.

While the present invention has been described with respect to the particular embodiments, it will be apparent to those skilled in the art that various changes and modifications may be made in the invention without departing from the spirit and scope thereof, as defined in the appended claims.

## Claims

1. A pharmaceutical or dietary supplement composition or food for special medical purposes comprising a therapeutically effective amount of palmitoylethanolamide or a pharmaceutically acceptable salt or derivative thereof, and a therapeutically effective amount of alpha-lipoic acid and at least one additional compound to be used for the prevention and/or treatment of pain and/or neuropathies.

2. The pharmaceutical or dietary supplement composition or food for special medical purposes according to claim 1, wherein the ratio of palmitoylethanolamide to alpha-lipoic acid is from 1: 0.5 to 1:4, preferably from 1:1 to 1:3 and most preferably 1:1.

3. The pharmaceutical or dietary supplement composition or food for special medical purposes according to claim 1 or 2, wherein said at least one additional compound is superoxide dismutase.

4. The pharmaceutical or dietary supplement composition or food for special medical purposes according to claim 1 2 or 3, wherein it further comprises B complex vitamins such as vitamin B1, vitamin B6 and vitamin B12

5. The pharmaceutical or dietary supplement composition or food for special medical purposes according to any preceding claim, wherein the amount of Palmitoylethanolamide in said composition is in the range of 20-40% by weight % of the total weight of the composition, and the amount of alpha-lipoic acid in said composition is in the range of 20-40% by weight % of the total weight of the composition.

6. The pharmaceutical or dietary supplement composition or food for special medical purposes according to claim 4, wherein it further comprises vitamin PP and vitamin E, and minerals, such as Magnesium and zinc

7. The pharmaceutical or dietary supplement composition or food for special medical purposes according to any preceding claim, wherein the PEA is in micronized or ultramicronized form.

8. The pharmaceutical or dietary supplement composition or food for special medical purposes according to any preceding claim, wherein it further comprises a gastroresistant or enteric film coating

9. The pharmaceutical or dietary supplement composition or food for special medical purposes according to claim 8, wherein said enteric coating comprises shellac, polyethylene glycol, hydroxypropyl methylcellulose, iron oxides and hydroxides, and titanium dioxide.

10. The pharmaceutical or dietary supplement composition or food for special medical purposes according to any preceding claim, wherein it further comprises pharmaceutically acceptable excipients selected from a group comprising of absorbents, glidants, diluents, binders, chelating agents, coating agents, pigments, complexing agents, solubilizers, emollients, emulsifiers, fillers, gelling agents, lubricants, moisturizers, retarding agents, stabilizers, suspending agents, sweeteners, thickening agents, surfactants, opacifiers, coloring agents, antigellants, rheology control agents, and their combinations thereof.

11. A process for the preparation of a pharmaceutical or dietary supplement composition or food for special medical purposes comprising a therapeutically effective amount of palmitoylethanolamide (PEA) or a pharmaceutically acceptable salt or derivative thereof, and a therapeutically effective amount of alpha-lipoic acid (ALA) and at least one additional compound to be used for the prevention and/or treatment of pain and/or neuropathies, wherein said process comprises following steps:
a) Mixing the individual active substances PEA, ALA and/or the at least one additional compound with tableting excipients such as binder, filler, lubricant and/or glidant in order to obtain uniform agglomeration;
b) Adding all said agglomerates of the active substances from step a) in a blender and mixing for appropriate time so as to form a homogenous mixture;
c) Sieving the obtained mixture of step b) and adding to the sieved mixture the total amount of at least one additional ingredient such as vitamins and/or minerals and at least one optional additional tableting excipient such as binder, diluent, lubricant and/or glidant and mixing for appropriate time until uniform;
d) Compressing the final blend from step c) into a desired tablet form, and
e) Optionally applying to the tablet a gastro-resistant or enteric film coating by any conventional method.

12. The process for the preparation of a pharmaceutical or dietary supplement composition or food for special medical purposes according to claim 11, wherein said at least one additional compound is superoxide dismutase.

13. The process for the preparation of a pharmaceutical or dietary supplement composition or food for special medical purposes according to claim 11, wherein said at least one additional ingredient is vitamins such as vitamins B complex, vitamin PP and vitamin E, and minerals, such as Magnesium and zinc.

14. The process for the preparation of a pharmaceutical or dietary supplement composition or food for special medical purposes according to claim 11, wherein said gastro-resistant or enteric coating comprises shellac, polyethylene glycol, hydroxypropyl methylcellulose, iron oxides and hydroxides, and titanium dioxide.
